# EUROPEAN PATENT APPLICATION

(11) **EP 4 365 274 A1**
(43) Date of publication of application: **08.05.2024**
(21) Application number: 22832252.5
(22) Date of filing: 29.06.2022
(51) Int. Cl.: C12M 1/00, C12M 1/36

(54) **ILLUMINATION DEVICE AND INCUBATOR FOR CONDUCTING MICROALGAE GROWTH EXPERIMENTS**

(30) Priority: 30.06.2021 ES 202130614
(71) Applicant: Universidad del País Vasco/Euskal Herriko Unibertsitatea, 48940 Leioa, Vizcaya (ES); Blanco Rayón, David, 48015 Bilbao (ES)
(72) Inventor: BLANCO RAYÓN, Esther, 48940 Leioa (ES); BLANCO RAYÓN, David, 48015 Bilbao (ES)
(74) Representative: Igartua, Ismael
(86) International application number: PCT/ES2022/070416
(87) International publication number: WO 2023/275423

(57) **Abstract**

A lighting device and incubator for microalgae growth experiments. The device comprises at least one light source (10) having leds (20) for illuminating microalgae and a control unit (30) configured to control the switching on and off of the leds (20) and to vary the light intensity of the leds (20). The light source (10) comprises RGB LEDs (20), and each RGB LED (20) has a housing that houses a red led (21), a green led (22) and a blue led (23) and a microcontroller (24) that is connected to the control unit (30) to vary the light intensity of the red led (21), the green led (22) and the blue led (23) of the RGB led (20), such that the colour and intensity of the light emitted by each RGB led (20) are modified by the control unit (30).

## Description

### TECHNICAL FIELD

The present invention relates to a lighting device for performing small and medium scale microalgae growth experiments in research laboratories, as well as an incubator incorporating the lighting device for performing microalgae growth experiments.

### PRIOR ART

In recent times, microalgae research has gained a lot of importance due to the different uses that can be given to them. Microalgae, among other applications, can be used to produce biofuels, pharmaceuticals, food, to treat wastewater or industrial water, for air conditioning and insulation of buildings, etc.

Microalgae cannot be harvested in large quantities directly from nature and must therefore be cultivated. For the different uses of microalgae to be economically viable, the production costs of microalgae need to be significantly reduced.

Currently, large-scale artificial cultivation of microalgae can be classified into open systems, mainly large ponds where microalgae are grown in the open air, and closed systems, where microalgae are grown inside photobioreactors, thus achieving a higher quality product. Regardless of the system employed, light is an essential factor for microalgae growth, and varies significantly with time of exposure to light, light intensity, and light spectrum. In open systems the light used is natural, and the low production rates are compensated by the large volumes of microalgae grown in the ponds, but in photobioreactors, which have a significantly smaller volume, an adequate artificial light source is required for microalgae growth.

The light demand required by microalgae for proper growth is much more complex than the light required by other types of plants, and varies significantly with each species of microalgae. For this reason, it is necessary to carry out small-scale experiments in research laboratories to determine the optimal conditions for the growth of each species of microalgae, so that these conditions can later be reproduced in photobioreactors on an industrial scale. Experiments are generally carried out with white light fluorescent lamps, and in some cases coloured filters are used to modify the colour of the light from the fluorescent lamps.

CN201746536U discloses a lighting device for performing microalgae growth experiments comprising a light source having leds for illuminating the microalgae and a control unit configured to control the switching on and off of the leds and to vary the light intensity of the leds. The lighting device is arranged in a temperature-controlled microalgae incubator in which the microalgae are placed. The incubator has an upper shelf with two led light sources and a lower shelf with fluorescent lamps, so that the incubator allows the difference in growth of microalgae illuminated with leds to be tested against microalgae illuminated with conventional fluorescent lamps. The light source comprises a panel with 100 high-brightness leds in red, green, blue, yellow and white, and the control unit varies the on/off and light intensity of the leds to simulate the gradual change of sunlight.

### DISCLOSURE OF THE INVENTION

The object of the invention is to provide a lighting device and an incubator for performing microalgae growth experiments as defined in the claims.

An aspect of the invention relates to a lighting device for performing microalgae growth experiments comprising at least one light source having leds for illuminating the microalgae and a control unit configured to control the switching on and off of the leds and to vary the light intensity of the leds. The light source comprises RGB LEDs, each RGB LED has a housing that houses a red led, a green led and a blue led and a microcontroller that is connected to the control unit to vary the light intensity of the red led, the green led and the blue led of the RGB LED, such that the colour and intensity of the light emitted by each RGB LED are modified by the control unit.

Another aspect of the invention relates to an incubator for performing microalgae growth experiments comprising shelves on which containers with the microalgae are placeable, at least one light source arranged on each shelf having leds to illuminate the microalgae, and a control unit configured to control the switching on and off of the leds and to vary the light intensity of the leds. The light source comprises RGB LEDs, each RGB LED has a housing that houses a red led, a green led and a blue led, and a microcontroller that is connected to the control unit to vary the light intensity of the red led, the green led and the blue led of the RGB LED, such that the colour and intensity of the light emitted by each RGB LED are modified by the control unit.

The control unit allows each RGB LED to be controlled independently from the light source, so that the switch-on time, the light intensity and the type of colour of each RGB LED can be modified. The RGB LEDs combine the primary colours red, green and blue to obtain any colour, which allows the microalgae to be illuminated with the required colour and intensity and for the required time, and therefore an infinite number of experiments can be carried out that can later be scaled up to an industrial level to cultivate microalgae in photobioreactors. For example, CN201746536U uses individual five-colour leds, so it cannot change the colour at the led level, which limits the experiments to the colours of the leds. Also, by using single-colour led, if a single colour wanted to be used, the leds of the other colours must be kept off, so CN201746536U uses high-brightness leds to achieve the required light intensity for microalgae growth.

These and other advantages and features of the invention will become apparent in view of the figures and the detailed description of the invention.

### DESCRIPTION OF THE DRAWINGS

Figure 1 shows a perspective view of the light source of the lighting device according to an example of the invention.
Figure 2 shows an exploded perspective view of the light source of the previous figure.
Figure 3 shows a schematic example of the electrical and data connections of the light source in Figure 1.
Figure 4 shows an incubator for microalgae growth experiments with light sources as shown in Figure 1.
Figure 5 shows an example of the arrangement of the light sources on a shelf of the incubator of Figure 4.
Figure 6 shows another example of the arrangement of the light sources on a shelf of the incubator of Figure 4.
Figure 7 shows a schematic example of the electrical and data connections of the light sources of the incubator of Figure 4.
Figure 8 shows the phycobiliprotein and biomass production of a cyanobacterial strain exposed to different colours with the lighting device.

### DETAILED DISCLOSURE OF THE INVENTION

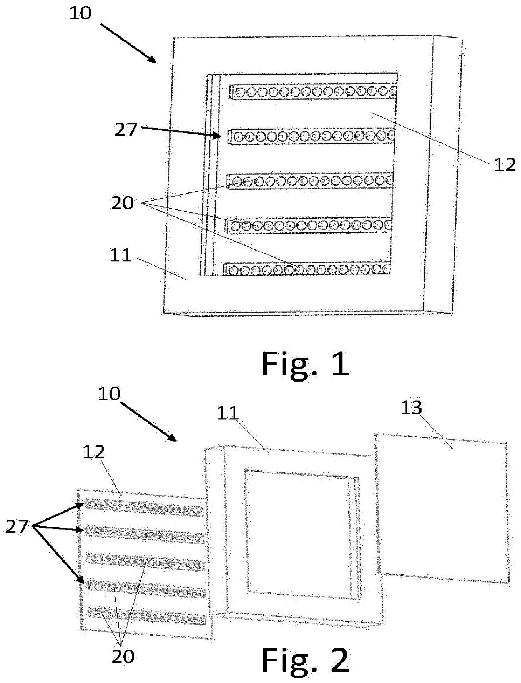

The invention relates to a lighting device for performing microalgae growth experiments comprising at least one light source 10 having leds 20 for illuminating the microalgae and a control unit 30 configured to control the switching on and off of the leds 20 and to vary the light intensity of the leds 20. See Figures 1 to 3.

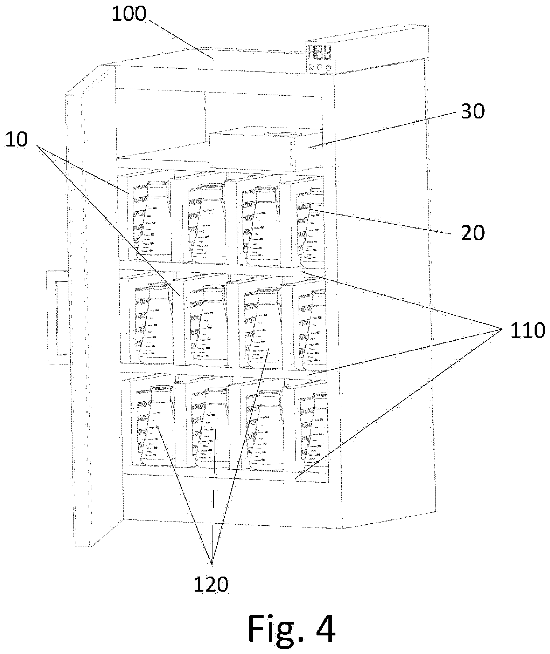

The lighting device is used to illuminate microalgae strains in a controlled environment for small-scale experiments to optimize the production of the microalgae strain or its products of interest. The lighting device can be installed in temperature-controlled rooms, incubators (as shown in Figure 4) or any other controlled environment that allows microalgae growth.

The light source 10 comprises RGB LEDs 20, wherein each RGB LED 20 has a housing that houses a red 21, green 22 and blue 23 led and a microcontroller 24 that is connected to the control unit 30 to vary the light intensity of the red 21, green 22 and blue 23 leds of the RGB LED 20, such that the colour and intensity of the light emitted by each RGB LED 20 are modified by the control unit 30.

In this way, the lighting device has as many microcontrollers 24 as there are RGB LEDs 20 in the light source 10, so that the control unit 30 can independently select for each RGB LED 20 the combination of red, green and blue colours to obtain the required colour, the light intensity of the RGB LED 20, and the switch-on time.

The lighting device has a power supply to electrically power the RGB LEDs 20 of the light source 10 and the control unit 30. The lighting device may have its own power supply, or a transformer and a conventional plug to connect to the grid. Preferably, the control unit 30 has the power supply integrated.

Figures 1 and 2 show an example of the light source 10. The light source 10 has a frame 11 supporting a rear cover 12 and a front diffuser 13, the rear cover 12 has a front face on which the RGB LEDs 20 are arranged and the front diffuser 13 is arranged on the front face of the rear cover 12 to homogenize the light emitted by the RGB LEDs 20.

The frame 11 defines a housing in which the rear cover 12 with the RGB LEDs 20 and the front diffuser 13 are housed, providing rigidity to the light source 10 for its correct transport, fixing and use. The rear cover 12 that supports the RGB LEDs 20 is made of an opaque material to direct the light towards the front diffuser 13. The front diffuser 13 is made of a translucent material that allows the light to pass through, homogenizing it and directing it towards the microalgae.

As can be seen in the figures, the RGB LEDs 20 are arranged in led strips 27 on the front face of the rear cover 12. Alternatively, the rear cover 12 with the RGB LEDs may be a single element, such as an RGB LED panel, or an RGB LED panel that is arranged on the rear cover 12.

Preferably, the led strips 27 extend parallel to each other and are arranged horizontally on the rear cover 12, so that a homogeneous and compact distribution of the RGB LEDs 20 is obtained, allowing the light to be concentrated.

Preferably, the lighting device further comprises an atmospheric sensor 40 for measuring the pressure, temperature and humidity of the environment in which the lighting device is arranged. The atmospheric sensor 40 is employed by the control unit 30 to monitor the environment in which the microalgae cultivation experiment is performed and to check that the pressure, temperature and humidity conditions are in accordance with the experiment. The atmospheric sensor 40 is a single device that integrates a pressure sensor, a temperature sensor and a humidity sensor. Alternatively, separate sensors can be used to measure the pressure, temperature and humidity of the environment.

Preferably, the lighting device further comprises at least one light sensor 50 for measuring the light intensity of the environment in which the lighting device is arranged. The light sensor 50 may be a photoresistor or LDR having a resistance that varies as a function of the light incident on the sensor. The light sensor 50 is employed by the control unit 30 to check that the light source 10 is correctly emitting light, and the control unit 30 may have an alarm management system to alert the user when the light source 10 is not illuminated according to the required light intensity.

Even more preferably, the lighting device further comprises a wireless data transmitter and receiver 60 that is associated with an external electronic device 70 for sending and receiving data of the status and switching on of each RGB LED 20, data of the light intensity and colour of each RGB LED 20, and for sending to the external electronic device 70 data of the light intensity, temperature, humidity, and pressure of the environment in which the lighting device is arranged. The wireless data transmitter and receiver 60 may be a modem with 4G connectivity.

The external electronic device 70 may be a computer, smartphone, tablet or the like with a custom-designed software application, through which the lighting device can be remotely controlled. The software application allows the parameterization of the light intensity and colour of each RGB LED 20, and allows programming the switch-on time of the light source 10, as well as the display of variables such as the temperature, pressure and humidity measured by the atmospheric sensor 40, or the light intensity measured by the light sensor 50.

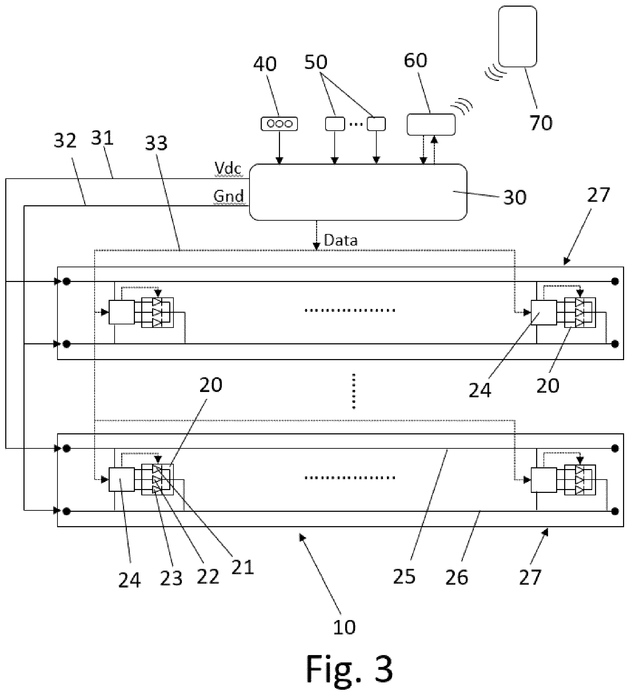

Figure 3 shows a non-limiting schematic example of the electrical and data connections of a lighting device as shown in Figures 1 and 2 with several led strips 27 of RGB LEDs 20. Each led strip 27 has several RGB LEDs 20, and each RGB LED 20 has a housing that houses a red led 21, a green led 22 and a blue led 23 and a microcontroller 24 that is connected to the control unit 30 to vary the light intensity of the red led 21, the green led 22 and the blue led 23 of the RGB LED 20. Each led strip 27 has power cables 25 and 26 which extend longitudinally along the led strip 27 and which are electrically connected to power cables 31 and 32 of the control unit 30. The control unit 30 has a data bus 33 represented with a dotted line which is connected to the microcontroller 24 of each RGB led 20 to control the timing and switching on of the RGB LEDs 20, their light intensity and colour type. The control unit 30 is operatively connected with the atmospheric sensor 40, the light sensors 50 and the wireless data transmitter and receiver 60 which is associated with the external electronic device 70.

The lighting device described above may comprise several light sources 10 controlled by the control unit 30. For example, the light sources may be arranged in a temperature-controlled room where there is a microalgae culture, or they may be arranged in a laboratory incubator as depicted in Figures 4 to 7.

Accordingly, the invention also relates to an incubator 100 for performing microalgae growth experiments comprising shelves 110 on which containers 120 with the microalgae are placeable, at least one light source 10 arranged on each shelf 110 having leds 20 to illuminate the microalgae, and a control unit 30 configured to control the switching on and off of the leds 20 and to vary the light intensity of the leds 20. The light source 10 comprises RGB LEDs 20, wherein each RGB LED 20 has a housing that houses a red led 21,a green led 22 and blue led 23 and a microcontroller 24 that is connected to the control unit 30 for varying the light intensity of the red led 21 ,the green led 22 and the blue led 23 of the RGB LED 20, such that the colour and intensity of the light emitted by each RGB LED 20 are modified by the control unit 30.

Preferably, on each shelf 110 are arranged lines 130 of containers 120, each line 130 having a left side and a right side, and each shelf 110 has rows 140 of light sources 10, with at least one light source 10 per row 140, and at least one side of each line 130 of containers 110 is illuminated by a respective row 140 of light sources 10. This ensures that all containers 120 on each shelf 110 are directly illuminated and that there are no containers 120 shading others.

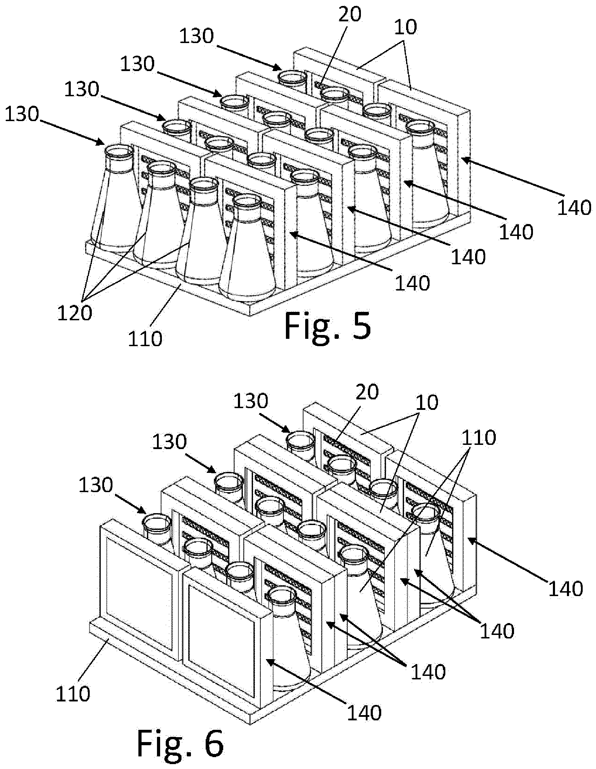

As shown in Figure 5, only one side of each line 130 of containers 110 is illuminated by a respective row 140 of light sources 10. Alternatively, and preferably, as shown in Figure 6, both left and right sides of each line 130 of containers 120 are illuminated by a respective row 140 of light sources 10. Some types of microalgae have a density that makes it difficult for light to pass through them, so rather than employing high brightness light sources 10 that can illuminate from only one side, it is preferable to employ light sources 10 that are lower in intensity but illuminate the containers 120 from both sides. In containers 110 that have a volume of 250 ml there is no problem for light to pass through, however, illuminating from both sides is useful when you want to increase the amount of light the crop receives to improve growth.

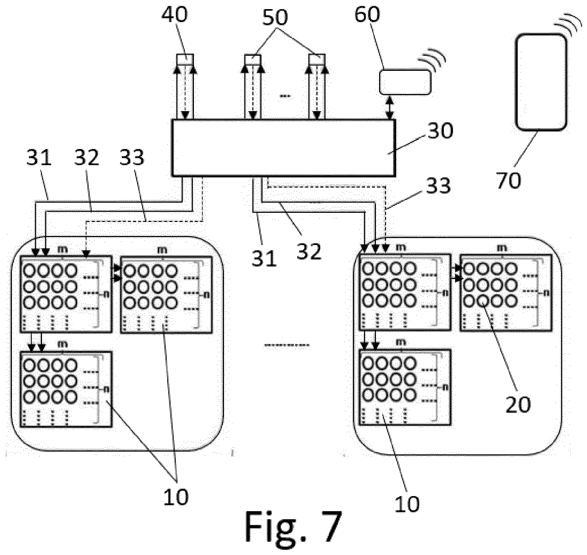

Figure 7 shows a non-limiting example of a lighting device as shown in Figures 1 and 2 with several led strips 27 of RGB LEDs 20. Each led strip 27 has several RGB LEDs 20, and each RGB LED 20 has a housing which houses a red led 21, a green led 22 and a blue led 23, and a microcontroller 24 that is connected to the control unit 30 for varying the light intensity of the red led 21,the green led 22 and the blue led 23 of the RGB LED 20. Each led strip 27 has power cables 25 and 26 extending longitudinally along the led strip 27, which are electrically connected to power cables 31 and 32 of the control unit 30. The control unit 30 has a data bus 33 represented with a dotted line which is connected to the microcontroller 24 of each RGB LED 20 to control the timing and switching on of the RGB LEDs 20, their light intensity and colour type. The control unit 30 is operatively connected with the atmospheric sensor 40, the light sensors 50 and the wireless data transmitter and receiver 60 which is associated with the external electronic device 70.

Figure 7 shows a non-limiting schematic example of the electrical and data connections of the power supplies 10 arranged on the shelves 110 of an incubator 100 as shown in Figure 4. The electrical and data connections in Figure 7 are identical to those depicted in Figure 3, but Figure 7 depicts the connection of several light sources 10.

Each light source 10 of the incubator has several RGB LEDs 20, and each RGB LED 20 has a housing that houses a red led 21, a green led 22 and a blue led 23, and a microcontroller 24 that is connected to the control unit 30 to vary the light intensity of the red led 21, the green led 22 and the blue led 23 of the RGB LED 20. The control unit 30 has power cables 31 and 32 for powering the light sources and a data bus 33 which is connected to the microcontroller 24 of each RGB LED 20 to control the timing and switching on of the RGB LEDs 20, their light intensity and colour type.

The incubator 100 comprises an atmospheric sensor 40 for measuring the pressure, temperature and humidity inside the incubator where the microalgae are available, and the control unit 30 is operatively connected with the atmospheric sensor 40.

The incubator 100 has a light sensor 50 on each shelf 110 to measure the intensity of each shelf 110 of the incubator, and the control unit 30 is operatively connected with the light sensors 50 to check that the light sources 10 on each shelf 110 are operating within the parameters required by the experiment.

The incubator further comprises a wireless data transmitter and receiver 60 that is associated with an external electronic device 70 for sending and receiving data of the status and power on of each RGB LED 20, data of the light intensity and colour of each RGB LED 20, and for sending to the external electronic device 70 data of the light intensity, temperature, humidity, and pressure of the incubator in which the microalgae are available.

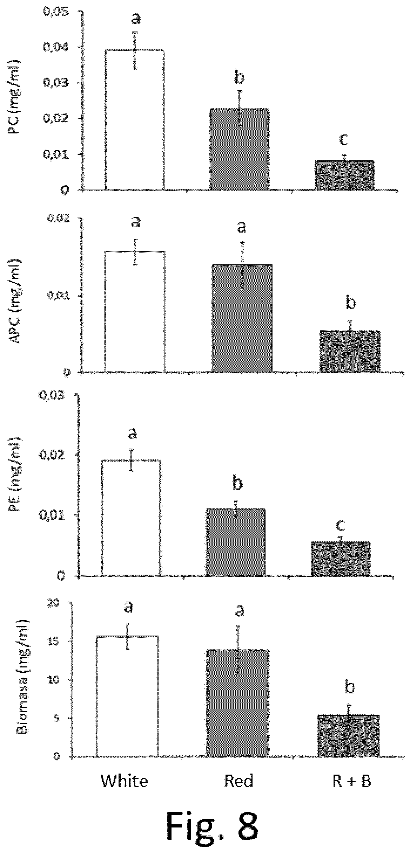

Figure 8 shows an experiment with the lighting device, where cyanobacteria were exposed to white light, red light and a combination of red and blue light. To obtain the white light, all the RGB 20 LEDs of the light source 10 were switched on in white light, to obtain the red light, all the RGB 20 LEDs of the light source 10 were switched on in red light, and to obtain the combined light, some of the RGB 20 LEDs were switched on in red light and some of the RGB 20 LEDs were switched on in blue light.

Specifically, the phycobiliproteins of a cyanobacterial strain (phycocyanin PC, allophycocyanin APC and phycoerythrin PE), as well as the biomass of the cyanobacterial strain, were exposed to white, red and the combination of red and blue light. As can be seen in the figure, the colour used has a great influence on relevant parameters in terms of microalgae production. It can be seen how white and red light achieved the best pigment and biomass production values in the strain studied. Accordingly, given these results, when cultivating this strain for industrial exploitation, the colour of the led source chosen for the photobioreactor would be red due to its lower energy consumption and its good results when producing the products of interest.

## Claims

1. Lighting device for performing microalgae growth experiments, comprising at least one light source (10) having leds (20) for illuminating the microalgae and a control unit (30) configured to control the switching on and off of the leds (20) and to vary the light intensity of the leds (20), **characterised in that** the light source (10) comprises RGB LEDs (20), and **in that** each RGB led (20) has a housing that houses a red led (21), a green led (22) and a blue led (23), and a microcontroller (24) that is connected to the control unit (30) to vary the light intensity of the red led (21), the green led (22) and the blue led (23) of the RGB LED (20), such that the colour and intensity of the light emitted by each RGB led (20) are modified by the control unit (30).

2. Lighting device according to claim 1, wherein the light source (10) has a frame (11) supporting a rear cover (12) and a front diffuser (13), the rear cover (12) has a front face on which the RGB LEDs (20) are arranged and the front diffuser (13) is arranged on the front face of the rear cover (12) to homogenize the light emitted by the RGB LEDs (20).

3. Lighting device according to claim 2, wherein the RGB LEDs (20) are arranged in led strips (27) on the front face of the rear cover (12).

4. Lighting device according to the preceding claim, wherein the led strips (27) extend parallel to each other and are arranged horizontally on the rear cover (12).

5. Lighting device according to any one of claims 1 to 4, further comprising an atmospheric sensor (40) for measuring the pressure, temperature and humidity of the environment in which the lighting device is arranged.

6. Lighting device according to any one of the preceding claims, further comprising at least one light sensor (50) for measuring the light intensity of the environment in which the lighting device is arranged.

7. Lighting device according to the preceding claim, further comprising a wireless data transmitter and receiver (60) that is associated with an external electronic device (70) for sending and receiving data of the status and switching on of each RGB LED (20), data of the light intensity and colour of each RGB LED (20), and for sending to the external electronic device (70) data of the light intensity, temperature, humidity, and pressure of the environment in which the lighting device is arranged.

8. Incubator for performing microalgae growth experiments, comprising shelves (110) on which containers (120) with the microalgae are placeable, at least one light source (10) arranged on each shelf (110) having leds (20) to illuminate the microalgae, and a control unit (30) configured to control the switching on and off of the leds (20) and to vary the light intensity of the leds (20), **characterised in that** the light source (10) comprises RGB LEDs (20), and **in that** each RGB LED (20) has a housing that houses a red led (21), a green led (22) and a blue led (23), and a microcontroller (24) that is connected to the control unit (30) to vary the light intensity of the red led (21),the green led (22) and the blue led (23) of the RGB LED (20), such that the colour and intensity of the light emitted by each RGB LED (20) are modified by the control unit (30).

9. Incubator according to the preceding claim, wherein the light source (10) has a frame (11) supporting a rear cover (12) and a front diffuser (13), the rear cover (12) has a front face on which the RGB LEDs (20) are arranged and the front diffuser (13) is arranged on the front face of the rear cover (12) to homogenize the light emitted by the RGB LEDs (20).

10. Incubator according to the preceding claim, wherein the RGB LEDs (20) are arranged in led strips (27) on the front face of the rear cover (12)

11. Incubator according to the preceding claim, wherein the led strips (27) extend parallel to each other and are arranged horizontally on the rear cover (12).

12. Incubator according to any one of claims 8 to 11, further comprising an atmospheric sensor (40) for measuring the pressure, temperature and humidity inside the incubator where microalgae are placeable.

13. Incubator according to any one of claims 8 to 12, further comprising a light sensor (50) on each shelf (110) for measuring the intensity of each shelf (110) of the incubator.

14. Incubator according to the preceding claim, further comprising a wireless data transmitter and receiver (60) which is associated with an external electronic device (70) for sending and receiving data of the status and switching on of each RGB LED (20), data of the light intensity and colour of each RGB LED (20), and for sending to the external electronic device (70) data of the light intensity, temperature, humidity, and pressure of the incubator where the microalgae are placeable.

15. Incubator according to any one of claims 8 to 14, wherein on each shelf (110) are arranged lines (130) of containers (120), each line (130) having a left side and a right side, and each shelf (110) has rows (140) of light sources (10), with at least one light source (10) for each row (140), and wherein at least one side of each row (130) of containers (120) is illuminated by a respective row (140) of light sources (10).

16. Incubator according to the preceding claim, wherein both left and right sides of each line (130) of containers (120) are illuminated by a respective row (140) of light sources (10).
